# EUROPEAN PATENT SPECIFICATION

(11) **EP 0 304 406 B1**
(45) Date of publication and mention of the grant of the patent: **28.06.1995**
(21) Application number: 88830340.1
(22) Date of filing: 11.08.1988
(51) Int. Cl.: G01N 33/18

(54) **Apparatus for automatically counting the microorganisms possibly present in liquids, particularly in waters for human use**
Vorrichtung, um möglicherweise in Flüssigkeiten vorkommende Mikroorganismen zu zählen, insbesondere in Wasser für den menschlichen Gebrauch
Appareil pour compter automatiquement les micro-organismes éventuellement présents dans des liquides, en particulier de l'eau pour la consommation humaine

(30) Priority: 13.08.1987 IT 4830587
(43) Date of publication of application: 22.02.1989
(73) Proprietor: MICROBO S.R.L., 00153 Roma (IT)
(72) Inventor: Antonini, Giovanni, Roma (IT); Valenti, Piera, Roma (IT)
(74) Representative: Massari, Marcello

(56) References cited:
- EP-A- 0 184 273
- US-A- 4 517 849
- PATENT ABSTRACTS OF JAPAN, vol. 7, no. 117 (P-198)[1262], 21 May 1983*

## Description

This invention refers to an apparatus for automatically counting the microorganisms possibly present in liquids, particularly in waters for human use.

As it is known, bacteriologic pollution of waters nowadays is so serious that the systems and methods used at present for detecting and controlling the bacterial charge of water are absolutely insufficient.

In fact, such systems and methods have many drawbacks, the temporal discontinuity of measurements and the delay existing between sampling, measurements and data analysis being particularly serious.

It is also known that the bacterial pollution of waters potentially more dangerous to human health is caused by bacteria of animal nature. Thus the possibility of readily and precisely detecting the number of total coliforms and fecal coliforms present in the water under control is particularly important for the use of waters. In fact, total and fecal coliforms are a sure sign of recent fecal contamination, while detection of fecal Streptococcus indicates old fecal contamination.

Accordingly, detection of these microorganisms is an essential parameter for defining the quality of waters. This is taken into account by the Italian Laws in force, such as Law n° 319 issued on 15.05.1976 "Control standard for protecting waters from pollution"; Law n°650 issued on 24.12.1979 "Integration and modifications to Laws n°17L/1973 and n°319/1976 concerning the protection of waters from pollution"; the Italian Decreto del Presidente del Consiglio dei Ministri (DPCM) issued on 04.02.1077 "General criteria, methods and technical standards according to art.2 letters b), d) and e) of Law n°318/1976"; the DPCM n°515 issued on 03.07.1982 "Enforcement of EEC directive n°75/440 concerning the quality of surface waters intended to be used for the production of drinkable water"; the DPCM issued on 08.02.1985 "Qualitative characteristics of water for human use".

The devices and methods used at present detect the presence and count the number of the microorganisms by discontinuous sampling and microbiological analysis of the water, using traditional methods.

These methods require a long time from the water sampling to the transport thereof to the laboratory where the microbiological analysis must be carried out and this may cause a modification in the original bacterial composition. On the other hand, an even longer time is normally required for culture and identification of microbic pollutants.

Finally, it can be said that at present the data on water microbic pollution can be obtained only after a period of time varying from 48 to 72 hours after water sampling. Furthermore, this temporal delay makes it impossible to early operate for eliminating or correcting the pollution causes.

Accordingly, it is an aim of the invention to overcome these drawbacks providing an apparatus intended to automatically analyze and measure the bacterial content of a liquids in a time varying from a few minutes to 12 hours, depending on the liquids bacterial charge, without involving a direct action of any operators.

Automatic devices for measuring bacteria content in water have been already described:

EP-A-0 184 273 to ERMA OPTICAL WORKS LIMITED (JP) discloses an apparatus for measuring impurities such as dust and bacteria in super-pure water by means of a sensor detecting voltage pulses caused by impurities passing trough a small hole.

JP-A-58 37559 to HOTSUKAIDOU KAIHATSUKIYOKU DOBOKU SHIKEN SHO (JP) discloses a continuous and automatic measuring device of bacteria wherein the test water is seeded to a culture fluid in a fermentation vessel. Thereafter the cultured water is sampled at a measuring section where bacterial growth is recorded by means of a turbidimeter or a spectrophotometer. In this apparatus the diluting and controlling devices, the fermentation tube and the measuring device are separated and the analysis requires several sampling steps.

The present invention is directed to an apparatus according to Claim 1.

The invention as defined in claims 1-10 will be now described in detail with reference to the annexed drawings, wherein:
Figure 1 is an overall view of the apparatus of the invention;
Figure 2 is a diametral sectional view of one of apparatus cells; and
Figure 3 is a 90 sectional view of the cells of Figure 2.

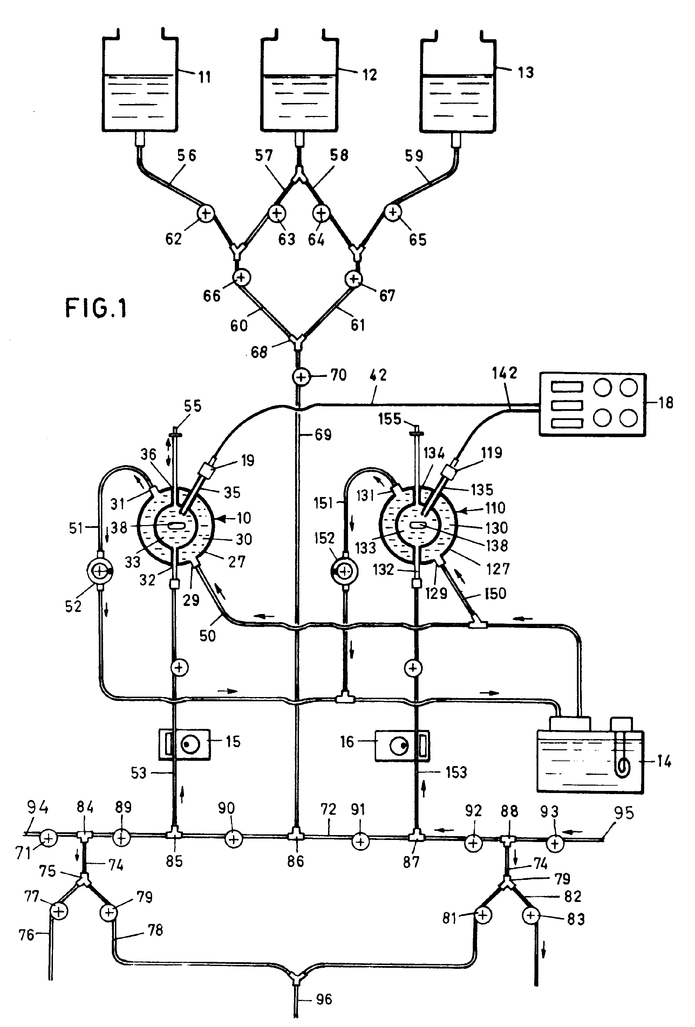

Firstly referring to Figure 1, the invention is shown therein comprising two cells, that is a measuring cell 10 and a control cell 110.

The apparatus also comprises:
a container 11 for the reactive solution to be mixed both with the water to be analyzed and with the control water;
a container 12 for the washing water;
a container 13 for a sterilizing substance, in this case sodium hypochloride, intended to reset the absolute sterile condition both of cell 10 and of cell 110;
a thermostatic device 14 connected both with cell 10 and will cell 110 for keeping a predetermined temperature therein;
a first peristaltic pump 15 intended to control the filling and emptying of cell 10 with the various liquids and reagents;
a second peristaltic pump 16 intended to control the filling and emptying of cell 110 with the various liquids and reagents;
an electric detector 18 connected both to a sensor 19 of cell 10 and to a sensor 119 of cell 110;
a series of solenoid valves controlled by a processor, and a piping system having the solenoids valves fitted thereon for connecting the various members of the apparatus.

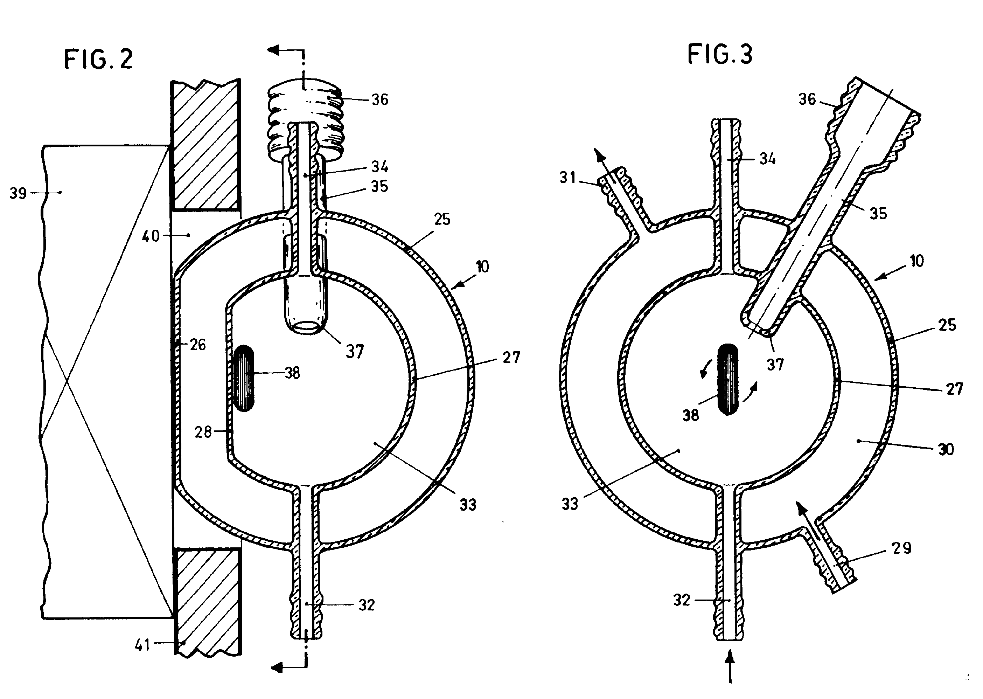

As is more clearly shown in Figures 1 and 2, cell 10 and cell 110 have the same structure, shape and size, each comprising:
a substantially spherical outer body 25 having a flat wall 26 parallel to the vertical diameter thereof; a concentric smaller inner body 27 having a flat wall 28 parallel to wall 26; a lower passage 29 connected to cavity 30 formed between body 25 and body 27; an upper passage 31 also connected to cavity 30; a lower vertical passage 32 crossing cavity 30 and connected to inner cavity 33 of inner body 27; an upper vertical passage 34 also connected to cavity 33 of body 27; a cylindrical sensor housing 35 crossing cavity 30 and having a threaded portion 36 out of body 25 and an open end 37 connected to cavity 33 of inner body 27.

Furthermore, cavity 33 of body 27 comprises an armature 38 magnetically operated by magnetic stirrer 39 placed in contact with wall 26 of cell 10 which is received within an opening 40 formed in the rear panel 41 of the apparatus supporting all the components thereof as a later described.

As mentioned above cell 110 is identical with the described cell 10 and Figure 2 the components thereof are referred to by the same reference numbers increased by 100.

Sensor housing 35 receives sensor 19 while sensor housing 135 receives sensor 119 (not shown in Figures 2 and 3), both sensor being connected to electric detector 18 through wires 42 and 142.

As shown Figure 1, in the embodiment of the invention comprising a single test cell and a single control cell, cell 10 and 110 are supported side by side on supporting rear panel 41 and they are operatively connected to the other members of the apparatus, as later described. cavities 30 and 130 of cells 10 and 110 are connected to thermostatic device 14 through input pipes 50 and 150 and output pipes 51 and 151 also connected to passages 29 and 129 and 31 and 131, respectively.

A flowmeter 52 is connected to pipe 51 while a similar flowmeter 152 is connected to pipe 151.

Lower passage 32 of cell 10 is connected to pipe 53 having a peristaltic pump 15 fitted thereon, while a sterile breather pipe filter 55 is fitted on upper passage 34.

Similarly lower passage 132 of cell 110 is connected to pipe 153 having a peristaltic pump 16 fitted thereof, while a sterile breather pipe filter 155 is fitted on upper passage 134.

As it is shown the solution container 11, the washing water container 12 and the sterilizing substance container 13 are connected to a piping referred to be references 56, 57, 58, 59, 60 and 61 controlled by solenoid valves 62, 63, 64, 65, 66 and 67 having an Y-shaped fitting 68 on the end thereof connected to a pipe 69 controlled by solenoid valve 70.

The other end of pipe 69 is connected to pipe 72 also connected to pipes 53 and 153.

Pipe 72 is also connected to a pipe 73 and a pipe 74. Pipe 73 is connected through an Y-shaped fitting 75 to a by pass 76 controlled by a solenoid valve 77 and to a discharge pipe 78 controlled by a solenoid valve 79.

Similarly, pipe 74 is connected through Y-shaped fitting 79 to pipes 80 and 82 controlled by solenoid valves 81 and 83.

The various couplings and connections are obtained through fitting 84,85,86,87,and 88, respectively.

Finally, pipe 72 is also connected to other six solenoid valves and more precisely:
- a solenoid valve 71 between fitting 84 and the test water sampling point 94;
- a solenoid valve 89 between fittings84 and 85;
- a solenoid valve 90 between fittings 85 and 86;
- a solenoid valve 91 between fittings 86 and 87;
- a solenoid valve 92 between fittings 87 and 88;
- a solenoid valve 93 between fitting 88 and the control water sampling point 95.

The apparatus of the invention operates as follows: the sterilizing substance of container 13 is fed to cells 10 and 110 to eliminate any microorganisms therefrom (step I) by suitably controlling solenoid valves 65, 67.70,90, and 91 in the open position and the other valves in the close position and operating peristalting pumps 15 and 16. After the necessary sterilization time (usually 5′ + 25′) the cells are emptied through discharge pipe 96 by inversely operating peristaltic pumps 15 and 16, opening solenoid valves 89, 79, 92 and 81 and closing all other solenoid valves (step II).

Then, a first washing of cells 10 and 110 is carried out by the water of container 12 opening solenoid valves 64, 67,70,90 and 91 and closing all other solenoid valves (step III).

A second washing is carried out opening solenoid valves 63, 66,70,90 and 91 and closing all other valves, after the discharge of the first washing water carried out as the discharge of the sterilizing substance (step II).

Also the second washing water is discharged from cells 10 and 110 repeating the emptying operations (step II) and the apparatus is thus ready to carry out the test operations, cell 10 and 110 being completely sterile since the water of container 12 is sterile. The test operations are carried out as follows: a given quantity of the reactive solution of container 11 is fed to cells 10 and 110 opening solenoid valves 62, 66, 70, 90, and 91 and operating peristaltic pumps 15 and 16 for a suitable time. Then, the water to be tested and the control water are fed to cells 10 and 110, respectively, opening solenoid valves 71, 89 and 93, 92 closing all other valves and operating peristaltic pumps 15 and 16.

It should be noted that during all the above-described sterilization, washing, test and control operation stirring armatures 38 and 138 have been operated by magnetic stirren 39 within cells 10 and 110.

Similarly, at the least during all the test and control operations inner cavities 33 and 133 of cells 10 and 110, respectively, will be kept at a constant temperature by circulating the water from thermostatic device 14 controlled by flowmeters 52 and 152 fitted on pipes 51 and 151 within outer cavities 30 and 130, respectively.

The mixing of the water to be tested with the reactive solution will cause chemico-physical variations of the mixture within cell 10 if any microorganisms are present, these variations detected by the sensor received within sensor housing 35, which sensor will feed signals corresponding to the possibly detected variations to electric detector 18.

All the sensor used in this invention are of a known type and, accordingly they will not be described herein.

Furthermore, it should be noted that all the above-described operations are controlled and timed by a logic also of a known type which needs not be described and illustrated.

The chemico-physical characteristics used for detecting the presence of microorganisms in cells 10 and 110 are the following:
a) the variation in the turbidity of the liquid due to the growth of microorganisms in suspension;
b) the modifications in the color of the substances added to the liquid due to the metabolic activity of the microorganisms ;
c) the alteration in the pH of the liquid due to the metabolic activity of the microorganisms ;
d) the alteration in the redox potential of reagents due to the products of the microorganisms metabolism;
e) the alteration in the quantity of the oxygen dissolved in the liquid due to the metabolic activity of the microorganisms;
f) the alteration in the quantity of the carbon dioxide dissolved liquid due to the metabolic activity of the microorganisms.

The known reagents and optical sensor or electrodes are indicated in the following examples:

### EXAMPLE N°1

Detection of the total bacterial charge of animal nature through the detection of the modification in time of the turbidity of the medium due to the multiplication of the microorganisms.

A concentrated nutrient solution containing glucose, yeast extract, peptone or any other nutrient adapted to the liquid containing the microorganisms.

After 30′ of lag phase the microorganisms start multiplying and clouding the culture liquid.

An optical reader based on the decrease of trasmittance fitted within the measurement cell, carries out the reading of turbidity at 620 Nm. The turbidity of the sample is related to the number of the bacteria per 1 ml of the sample by the control system.

### EXAMPLE N°2

Detection of the bacterial charge of coliforms through the detection of the modification in time of the turbidity of the medium due to the selective multiplication of coliforms by using a suitable coliform selective nutrient medium.

A concentrated nutrient solution comprising the so called Mac Conkey's medium containing lactose, cresol bromine, peptone, oxgall, suitable for the development of Escherichia coli and inhibiting the development of gram positive bacteria is added to the liquid containing E.coli.

The turbidity of the sample is measured by optical readers fitted in the measurement cells reading at 620 Nm.

The charge in the turbidity of the sample is related to the number of E.coli per 1 ml of the sample by the control system.

### Example N° 3

Detection of the quality of microorganisms and their partial characterization trough the detection of the modification in time of the color of a specific dying substance added to a suitable selective nutrient medium.

It is known that most strains of E.coli are able to ferment the lactose thus producing acids and gas. The use of the above-described Mac Conkey's medium reveals the presence of lactose fermenting bacteria trough the color charge due to the cresol bromine red.

### EXAMPLE N° 3 bis

On the other hand, the presence of bacteria of the kind of Streptococcus faecalis can be detected using a medium comprising peptone, dextrose, dipotassic,phosphate,monopotassic phosphate sodium azide,, cresol bromine red, pH 6.9.

The charge in color due to the metabolic activity of the microorganisms is detected by a colorimeter and related to the number of E.coli or Streptococcus faecalis per 1 ml of the sample by the control system.

### EXAMPLE N° 4

Detection of the quantity of the microorganisms trough the detection of the modification in time of the medium pH due to the metabolic activity of the microorganisms in a suitable selective medium.

As a mentioned above in example n° 3 the growth of E.coli on a lactose causes the production of acids detected by a decrease in the pH.

The detection trough an electrode of the variation in the pH in a nutrient medium comprising lactose indicates the presence of bacteria the metabolism of which is controlled in time and related to the number of E.coli per 1 ml of the sample by the control system.

### EXAMPLE N° 5

Detection of the quantity of microorganisms through the detection of the modification in time of the liquid redox potential by using redox mediators able to be metabolized by the bacteria.

When the removal of a pair of electrodes or atoms of hydrogen from an organic substrate is accompanied by the reduction of oxygen to water, an exchange of free energy takes place a portion of which is transformed into adenosinetriphosphate.

The adenosinetriphosphate produced is continuously measured trough the well-known dosage of luciferin. The dosage of luciferin is related to the number of bacteria per 1 ml of the sample by the control system.

### EXAMPLE N°6

Detection of the quantity of microorganisms through the detection of the modification in time of the quantity of dissolved oxygen by using a specific electrode and reducing substances able to be metabolized by the microorganisms.

A solution containing sodium ascorbate, thymidylic acid, glucose, sucrose, valinomycin is added to the liquid containing the microorganisms.

The dissolved oxygen is detected by an oxygen electrode.

The oxygen consumption due to the oxidation of the reagents caused by the metabolism of the bacteria is controlled in time and related to the number of bacteria per 1 ml of the sample by the control system.

### EXAMPLE N.7

Detection of the quantity of microorganisms through the detection of the modification in time of the quantity of dissolved carbon dioxide by means of a specific electrode and substances able to be metabolized into carbon dioxide.

A solution containing peptone, dextrose, dipotassic phosphate, monopotassic phosphate, sodium azide is added to the liquid containing the microorganisms.

The detection of the carbon dioxide content is carried out by means of a CO₂ electrode and its modification in time is related to the number of bacteria per 1 ml of the sample by the control system.

It is intended that the above mentioned and described reagents and sensors can be varied and/or modified according to the microbic strain to be detected the construction and operation of the apparatus of the invention being the same.

Furthermore, it is intended that the invention which has been described and illustrated with reference to one particular embodiment thereof for testing waters for human use, can be used for detecting the presence, the number and the strain and/or the microbic kind of microorganisms in liquid solutions of any type.

As far as the operation is concerned the apparatus of the invention can be adjusted by suitably operating on the control logic thereof to carry out the test operation in cell 10 and the control operation in cell 110 at different times repeating the operation on the test water after a time predetermined by the measurement carried out by cell 10.

Furthermore, the apparatus can be used for example using cells 10 and 110 to control the water of an aqueduct in its "native" state and after chlorination and the like.

It should be noted that the whole apparatus is received within a conditioned container.

As a further characteristic of the invention it should be noted that when it is used to control natural waters such as watercourses and the like it is possible to create a continuous circulation of the test water in pipes 75 and 75 by suitably opening solenoid valves 71 and 77, thus allowing the conditions of the apparatus to be as similar as possible to the conditions of such water.

## Claims

1. An apparatus for automatically counting the microorganisms possibly present in liquids, particularly but not exclusively in waters for human use, comprising: a means (94;95) for sampling the liquid to be tested; a means (53;153) for drawing said liquid into fermentation cells (10;110) each of said cells containing a sensor means (19;119) adapted to detect the chemico-physical modifications of the liquid to be tested-nutrient solution mixture due to the presence of microorganisms therein and to transmit signals corresponding to the intensity of said variations due to the presence of microorganisms; a plurality of pipes (53,56,57,58,59,60,61,69,72,74,76,78,82,94,95,96,153) and solenoid valves (62,63,64,65,66,67,70,71,77,79,81,83, 89, 90,91,92,93) fitted thereon for selectively connecting the various operative means; an electronic synchronization and control means (18) for operating said various operative means and controlling the operation thereof; and a control and storing means (18) adapted to receive the analytical signals; this apparatus being characterized in that it comprises in combination: at least two cells, one of which (10) is feeded with the liquid to be analyzed for The microorganisms content and the other (110) with control water, said cells being made of a suitable material, preferably glass, comprising an outer body (25) of a substantially spherical shape having a flat wall (26) parallel to the vertical diameter thereof, an inner body (27) also having a substantially spherical shape and concentrically positioned relative to said outer body (25) and having a flat wall (28) parallel to said flat wall (27) of said outer body (25), a lower passage (29) and an upper passage (31) connected to the cavity (30) formed between said two bodies (25,27), and upper vertical passage (34) and a lower vertical passage (32) crossing said cavity (30) and being connected to said inner body (27) and a cylindrical housing (35) crossing said cavity (30) and having one end (36) out of said outer body (25) and a second open end (37) within said inner body (27), said cylindrical housing (35) receiving one said sensor (19;119); a thermostatic means (14;50,51,52;150,151,152) adapted to keep the temperature of the said mixtures contained in said cells at a constant predetermined value; a stirring means (38,39;138,139) adapted to mix the liquids to be tested with the nutrient solutions within said cells; a means (11) for containing a nutrient solution, a means (12) for containing a washing liquid; a means (13) for containing a sterilizing substance; a means (56,57,58,59,60,61) for drawing the nutrient solution, the washing liquid and the sterilizing liquid; an analytical means (18) adapted to compare the said signal coming from the said cell (10) feeded with the liquid to be analyzed with the said signal coming from the said cell (110) feeded with control water and to correlate said signals with the number of microorganisms present in said cells (10;110).

2. The apparatus according to claim 1, wherein a peristaltic pump (15;16) is used for drawing said nutrient solution, said washing liquid, said sterilizing substance and said liquid to be tested.

3. The apparatus according to claim 2, wherein said peristaltic pump (15;16) is connected to said pipes (53, 56,57,58,59,60,61,69,72,74,76,78,82,94,95,96,153) so as to selectively feed said liquids to be tested, said nutrient solution , said sterilizing substance and said washing liquid to said cells (10;110), according to the opening and closing of said solenoid valves (62,63,64,65,66,67,70,71,77,79,81,83,89,90,91,92,93).

4. The apparatus according to claim 1, wherein said sensor means (19;119) comprises optical readers.

5. The apparatus according to claim 1, wherein said sensor means (19;119) comprises colorimeters.

6. The apparatus according to claim 1, wherein said sensor means (19;119) comprises a pH measuring means.

7. The apparatus according to claim 1, wherein said sensor means (19;119) comprises a turbidity measuring means.

8. The apparatus according to claim 1, wherein said sensor means (19;119) is a means for measuring the modification of the redox potential.

9. The apparatus according to claim 1, wherein said sensor means (19;119) comprises an oxygen electrode.

10. The apparatus according to claim 1, wherein said sensor means (19;119) comprises a carbon dioxide electrode.

## Patentansprüche

1. Ein Apparat zur automatischen Auszaehlung von Mikroorganismen, die moeglicherweise anwesend sind in Fluessigkeiten, besonders, aber nicht ausschliesslich, in Gewaessern fuer den menschlichen Gebrauch, der die Mittel enthaelt (94;95) zur Entnahme der zu testenden Fluessigkeit, die Mittel, enthaelt (53;153) zur Einfloessung der gesagten Fluessigkeit in den Fermentierungszellen (10;110), jede der genannten Zellen enthaelt einen Sensor (19;119), welcher dazu geeignet ist, die chemisch-physikalischen Veraenderungen der Mischung, bestehend aus der zu testenden Fluessigkeit und der Nahrungs-Losung, welche auf das Vorhandensein der darin enthaltenen Mikroorganismen zurueckzufuehren sind, zu entdecken und Signale wiederzugeben, welche der Intensitaet der gesagten Variationen entsprechen, die auf das Vorhandensein der Mikroorganismen zurueckzufuehren sind; bestehend aus einer Mehrzahl von Schlaeuchen (53;56;57;58;59;60;61;69;72;74;76;78;82;94;95;96;153) und solenoiden Ventilen (62;63;64;65;66;67;70;71,77,79;81,83,89,90;91;92;93), die daran befestigt sind, um die verschiedenen Funktionen selektiv zu verbinden; bestehend aus einer elektronischen Synchronisations- und Kontrollvorrichtung (18), zur Bedienung der genannten verschiedenen Funktionsvorrichtungen und zur Kontrolle, der darauffolgenden Operationen; bestehend aus einer Kontroll- und Speichervorrichtung (18), die dazu geeignet ist, analytische Signale zu empfangen; dieser Apparat ist dadurch charakterisiert, dass er kombiniert folgende Merkmale aufweist: mindestens zwei Zellen, wovon eine (10) mit der auf den Inhalt von Mikroorganismen hin zu testenden Fluessigkeit gefuellt wird, waehrend die andere (110) mit sterilem Wasser gefuellt ist, die genannten Zellen, aus einem geeignetem Material gearbeitet, vorzugsweise aus Glas, bestehen aus einem aeusseren Koerper (25) mit etwa sphaerischer Form mit einer flachen Seite (26) parallel zum vertikalen Querschnitt, einem inneren Koerper (27) auch von etwa sphaerischer Form und konzentrisch zu gesagtem aeusserem Koerper (25) angeordnet, mit einer flachen Seite (28) - welche parallel zur bereits genannten flachen Seite (27) des aeusseren Koerpers (28) ist, einem oberen (29) und einem unteren (31) Durchgang in Verbindung mit dem Zwischenraum (30) der zwei genannten Koerper (25;27), einem oberen vertikalen Durchgang (34) und einem unteren vertikalen Durchgang (32), die, gesagten Zwischenraum (30) durchqueren und die verbunden sind mit dem genannten inneren Koerper (27) und einem zylindrischen Behaelter (35), welcher den genannten Zwischenraum durchquert (30) und das eine Ende (36) im genannten aeusseren Koerper (25) und das andere offene Ende (37) im gennanten zentralen Koerper (27) hat, der genannte zylindrische Behaelter (35) enthaelt den genannten Sensor (19;119);
er bestehet aus einer thermostatischen Vorrichtung (14;50;51;52;150;151;152), welche die Temperatur der genannten Mischungen in den genannten Zellen konstant auf einen vorbestimmten Wert halten soll; er bestehet aus einer Ruehrvorrichtung (38;39;138;139), dazu geeignet, die zu testenden Fluessigkeiten mit der Nahrungs-Loesung in den gennanten Zellen zu mischen; er bestehet aus einer Vorrichtung (11), welche eine Nahrungs-Loesung enthaelt; er bestehet aus einer Vorrichtung (12), welche eine Spuehlungs-Loesung enthaelt; er bestehet aus einer Vorrichtung (13), welche ein sterilisierendes Mittel enthaelt; er bestehet aus einer Verbindung (56;57;58;59;60;61) der Nahrungs-Loesung, der Spuehlungs-Loesung und des sterilisierenden Mittels; er bestehet aus einer analytischen Vorrichtung 18, welche dazu geeignet ist, einer Vergleich anzustellen zwischen dem genannten Signal der genannten Zelle (10) , die mit der zu testenden Fluessigkeit gefuellt ist und dem genannten Signal der genannten Zelle (110), die mit der sterilen Fluessigkeit gefuellt ist, und dazu geeignet ist die genannten Signale in Verbindung zu bringen mit der Zahl der Mikroorganismen, die in den genannten Zellen vorhanden sind (10;110).

2. Der Apparat, entsprechend Patentanspruch 1, worin eine peristaltische Pumpe (15;16) verwendet wird um die Nahrungs-Loesung, die Spuehlungs-Loesung und das sterilisierende Mittel und die genannte zu testende Fluessigkeit zu transportieren.

3. Der Apparat, entsprechend Patentanspruch 1, worin die genannte peristaltische Pumpe (15;16) verbunden ist mit den genannten Schlaeuchen (53;56;57;58;59;60;61,69;72;74;76;78;82;94;95;96;153), um die genannten Zellen (10;110) selektiv zu fuellen mit der genannten zu testenden Fluessigkeit, mit der genannten Nahrungs-Loesung, mit dem genannten sterilisierenden Mittel und der genannten Spuehlungs-Loesung , in Verbindung mit dem Oeffnen und Schliessen der genannten solenoiden Ventilen (62;63;64;65;66;67;70;71;77;79;81;83;89;90;91;92;93).

4. Der Apparat, entsprechend Patentanspruch 1, worin der genannte Sensor (19;119) Teil einer optischen Lesevorichtung ist.

5. Der Apparat, entsprechend Patentanspruch 1, worin der genannte Sensor (19;119) Teil eines Kolorimeters ist.

6. Der Apparat, entsprechend Patentanspruch 1, worin der genannte Sensor (19;119) Teil einer Vorrichtung zur Messung des pH-Wertes ist .

7. Der Apparat, entsprechend Patentanspruch 1, worin der genannte Sensor (19;119) Teil einer Vorrichtung zur Messung der Truebung ist .

8. Der Apparat, entsprechend Patentanspruch 1, worin der genannte Sensor (19;119) Teil einer Vorrichtung zur Messung der Variation des Redox Potentiales ist.

9. Der Apparat, entsprechend Patentanspruch 1, worin der genannte Sensor (19;119) Teil einer Oxygen Elektrode ist .

10. Der Apparat, entsprechend Patentanspruch 1, worin der genannte Sensor (19;119) Teil einer Kohlenstoffdioxyd Elektrode ist .

## Revendications

1. L'appareil pour les comptes automatiques des microorganismes eventuellement prèsents dans des liquides, particulièrement mais non exclusivement dans l'eau pour l'utilisation humaine, comprenant: moyens (94;95) pour essayer le liquide qui doit être analysé; moyens (53;153) pour prélever le liquide en question dans les récipients (10;110) de fermentation, chaque récipient contenant un senseur (19;119) adapté pour determiner les modifications chimiques et physiques du mèlange du liquide-solution nutritive qui doit être assayé, provoquées par la presence de microorganismes à l'intérieur et pour transmettre les signaux correspondant à l'intensité de ces variations causées par la prèsence des microoganismes; plusieurs tubes (53;56;57;58;59;60;61;69;72;74;76;78;82;94;95;96;153) inserés dans les soupapes solénoides (62;63;64;65;66;67;70;71;77;79;81;83;89;90;91;92;93) pour réunir selectivement les different moyens opératifs; une synchronisation électronique et des moyens de contrôle (18) pour le fonctionnement des different moyens opératifs et pour le contrôle des opérations citées; et un contrôle et des moyens (18) pour emmagasiner adaptés pour recevoir les signaux analytiques; cet appareil est caractérisé par: au moins deux récipiants desquels un (10) est alimenté par un liquide à analyser pour son contenu en microorganimes et l'autre (110) par de l'eau sterile, ces récipients étant construits avec des matériaux convenables, de prefèrence en verre, comprenant un autre corps (25) avec une forme essentiellement sférique et un côté plat (26) parallel au diamètre vertical, un corps intérieur (27) ayant lui aussi une forme essentiellement sférique et placé concentriquement à l'autre corps mentionné (25) et ayant un côté plat (28) parallel au côté plat (27) mentionné de l'autre corps (25), un passage supérieur (29) et un inférieur (31) uni à la cavité (30) presenté entre les corps mentionnés (25;27), et un passage vertical supérieur (34) et un passage vertical inférieur (32) qui traverse la cavité mentionnée (30) et étant unie au corps (27) intérieur et un canal cilindrique (35) qui traverse la cavité mentionnée (30) et ayant une partie finale (36) au dehors du corps exterieur mentionne (25) et une deuxiéme ouverture terminale (37) et dans le corps intérieur (27), une canal cilindrique (35) qui reçoit un senseur (19;119); un système de thermostatation (14;50;51;52;150;151;152) pour vérifier la témperature des mélanges mentionnés contenus dans les récipients à une valeur costante predeterminée; un moyen d'agitation (38;39;138;139) propre pour mélanger les liquides qui doivent être assayés avec les solutions nutritives a l'intérieur des récipients mentionnés; un moyen (11) pour contenir la solution nitritive, un moyen (12) pour contenir le liquide de nettoyage; une moyen (13) pour contenir le liquide de sterilisation; moyens (59;57;58;59;60;61) pour prelever la solution nutritive, le liquide de nettoyage et le liquide de sterilisation; un moyen analytique (18) propre pour comparer le signal mentionné dérivant du recipient (10) mentionné alimenté avec le liquide a analyser avec le signal mentionné qui derive du récipient mentionné (110) alimente avec de l'eau sterile et pour correler les signaux en question avec le nombre de microorganismes présents dans les récipients mentionnés (10;110).

2. L'appareil en accord avec la revendication 1, possède une pompe peristaltique (15;16) utilisée pour prelever la solution nutritive mentionnée, le liquide de nettoyage mentionné, le compose' à steriliser et le liquide mentionné à analyser.

3. L'appareil en accord avec la revendication 2, posséde une pompe peristaltique (15;16) unie avec les tubes mentionnés (53;56;57;58;59;60;61;69;72;74;76;78;82;94;95;96;153) pour alimenter selectivement les liquides à analyser, la solution nutritive mentionnée, le compose' à steriliser et le liquide de nettoyage mentionné pour les recipients (10;110) en question, en accord avec l'ouverture et la fermeture des soupapes solénoides (62;63;64;65;66;67;70;71;77;79;81;83;89;90;91;92;93).

4. L'appareil en accord avec la revendication 1, posséde un senseur (19;119) comprenant un lecteur optique.

5. L'appareil en accord avec la revendication 1, posséde un senseur (19;119) colorimetrique.

6. L'appareil en accord avec la revendication 1, posséde un senseur (19;119) pour mesurer le pH.

7. L'appareil en accord avec la revendication 1, posséde un senseur (19;119) pour mesurer la turbidité.

8. L'appareil en accord avec la revendication 1, posséde un senseur (19;119) pour mesurer la modification du potentiel redox.

9. L'appareil en accord avec la revendication 1, posséde un senseur (19;119) avec un éléctrode pour l'oxygene.

10. L'appareil en accord avec la revendication 1, posséde un senseur (19;119) avec un électrode pour l'anydride carbonique.
